# EUROPEAN PATENT SPECIFICATION

(11) **EP 1 056 766 B1**
(45) Date of publication and mention of the grant of the patent: **15.08.2007**
(21) Application number: 99956692.0
(22) Date of filing: 26.10.1999
(51) Int. Cl.: C12Q 1/70

(54) **DETECTION OF HUMAN PAPILLOMA VIRUS IN PAPANICOLAOU (PAP) SMEARS**
NACHWEIS DES HUMANEN PAPILLOMA-VIRUS MIT HILFE DES PAPANICOLAU-TESTS
DETECTION DE PAPILLOMAVIRUS HUMAIN PAR LE TEST DE PAPANICOLAOU (PAP)

(30) Priority: 26.10.1998 US 105657 P
(43) Date of publication of application: 06.12.2000
(73) Proprietor: VENTANA MEDICAL SYSTEMS, INC., Tucson, AZ 85737 (US)
(72) Inventor: LIGHT, Elizabeth, S., Gaithersburg, MD 20882 (US); NUOVO, Gerard, Westerville, OH 43081 (US)
(74) Representative: Müller-Boré & Partner Patentanwälte
(86) International application number: PCT/US1999/025109
(87) International publication number: WO 2000/024760

(56) References cited:
- US-A- 5 283 171
- US-A- 5 484 699
- US-A- 5 656 423
- US-A- 5 679 509
- US-A- 5 712 092
- US-A- 5 783 412
- JACOBS ET AL.: "A general primer GP5+/GP6+-mediated PCR-Enzyme-Immunoassay method for rapid detection of 14 high-risk and 6 low-risk human papillomavirus genotypes in cervical scrapings" JOURNAL OF CLINICAL MICROBIOLOGY, vol. 35, no. 3, March 1997 (1997-03), pages 791-795, XP002241732
- JACOBS ET AL.: "Group-specific differentiation between high and low-risk human papillomavirus genotypes by general primer-mediated PCR and two cocktails of oligonucleotide probes" JOURNAL OF CLINICAL MICROBIOLOGY, vol. 33, no. 4, 1995, pages 901-905, XP002241733
- SCHIFFMAN ET AL: "Accuracy and interlaboratory reliability of human papillomavirus DNA testing by hybrid capture" JOURNAL OF CLINICAL MICROBIOLOGY, vol. 33, no. 3, March 1995 (1995-03), pages 545-550, XP002241734
- NUOVO: "Detection of human papillomavirus in Papanicolaou smears: correlation with pathologic findings and clinical outcome" DIAGN. MOL. PATHOL., vol. 7, no. 3, 1998, pages 158-163, XP008017385
- HIRT ET AL.: "Nucleotide sequence of human papillomavirus (HPV) type 41: an unusual HPV type without a typical E2 binding site consensus" VIRUS RESEARCH, vol. 18, no. 2-3, 1991, pages 179-189, XP008017383

## Description

The present invention relates to methods and reagents to detect viral DNA in atypical PAP smears indicative of predisposition for cancer.

Human Papilloma Virus (HPV) is the most common sexually transmitted viral disease and is manifested in over 1 million people per year in the United States. There are at least 90 distinct types of HPV of which over 25 are found in the lower genital tract. Of the HPV types found in genital lesions, some are implicated in cervical precancers and cancers.

These types are ranked according to a high, intermediate or low risk or tendency to progress to cancer. Common high risk types include: 16, 18, 39, 45, 56 etc., intermediate risk types include: 31, 33, 35, 51, 52, 58 etc., and low risk types include: 6, 11, 42, 43, 44 etc.

Human Papilloma Virus (HPV) is a family of DNA viruses where the genome is about 7900 base pairs having seven open reading frames expressed early in infection and two expressed late in infection. Protein E6 and E7 are linked to inducing transformation of benign cells in vitro and in vivo. While the exact mechanism is yet to be proven, one of the proteins from high-risk strains appears to inactivate the human tumor suppressor gene p53.

Currently, Pap smears are performed yearly on women to check for a presence of atypical or cancerous cells. Roughly 90% of all smears are normal, 3% are unequivocally dysplastic including LSIL and 7% are squamous atypias (ASCUS). Patients with a diagnosis of dysplasia are brought back to their doctor's office for biopsy and further procedures. Normal results are untreated. The ASCUS and LSIL diagnoses present the doctor and patient with multiple choices for treatment. If these patients could be tested for high risk type HPV presence it would provide further information on the best treatment route. The presence of a low risk HPV type could indicate no further action except perhaps more frequent pap smears. A high risk HPV type presence could indicate a more aggressive approach: either a cone biopsy, LEEP (loop electrode excision procedure), or ablative therapy (laser surgery or cryotherapy).

An alternative to standard Pap smears is the Cytyc Corporation's ThinPrep test. A standard Pap smear involves sampling the uterine cervix with a spatula or cytobrush and smearing the cells directly onto a glass slide. The ThinPrep test involves suspending the sample in a buffered fixative solution which provides better sample recover, fewer artifacts, multiple slides from one sample, less cell crowding, overlap and better cellular morphology.

In a ThinPrep sample, the sampling device is rinsed into a buffered preservative solution and this solution is used to make slides. By suspending the sample in preservative solution and using the ThinPrep machine to make the slides, the resulting slide is a thin-layer preparation that is cleaner of blood, inflammatory cells, mucus, and other obscuring artifacts than a standard Pap smear. The FDA has found the ThinPrep test to be preferable to the standard Pap smear for detection of LSIL and more severe lesions. Another advantage of the ThinPrep test is that the entire sample is not used to make a single slide. The preserved cells can be used to make more slides on which HPV testing could be performed.

The standard current methods for detecting cervical cancer is the PAP smear or Cytec's ThinPrep. Both detect atypical or cancerous cells, not HPV infection. PAP smears are classified as:
- normal
- ASCUS (atypical cells of uncertain significance)
- LSIL (low-grade squamous intraepithelial lesion)
- HSIL (high-grade squamous intraepithelial lesion)

Currently about 4.4 million PAP smears are classified as ASCUS per year. 2.5 million PAP smears are classified as LSIL per year. This indicates that 6.9 million or 7-8% of all PAP smears per year are ambiguous in their results. Knowledge of which HPV type present would aid the patient and clinician in determining risk level and treatment options.

Patients with an ambiguous cytology may still have preinvasive or microinvasive cancer and HPV DNA typing may aid in differentiating patients. Studies have shown that ambiguous cytology and high risk HPV infection with types 16, 18 and 31 are more likely to have high grade SIL or microinvasive histopathology on biopsy. Studies have also suggested that acute infection with HPV types 16 and 18 confer an 11 to 16.9 fold risk of rapid development of CIN.

Accordingly, a need exists to differentiate the borderline Pap smears and ThinPrep slides based on the HPV type present, if any. Other attempts at preparing oligonucleotide probes have been reported. See U.S. Patents 5,679,509, 5,057,411, Cole et al, Journal of Molecular Biology 193: 599-608 (1987), 5,554,538, 5,484,699, 5,501,947, 4,849,332, 5,411,847, 4,908,306, EP 0,477,972 B1, 5,527,898, 4,983,728, 4,849,334, 4,849,311 and 4,820,530. Applicants are also aware of U.S. Patent 5,538,871. However, each oligonucleotide probe appears to be specific to only one type, thus, many would be needed to form a complete set to detect all high risk HPV types.

Nuovo, Diagn. Mol. Path.,1998, Vol. 7, No. 3, pages 158-163, describes a study for determining the conditions needed for optimal HPV detection by in situ hybridization in archival Pap smears and to correlate the viral findings and pathological results and, for certain patients, clinical follow-up.

Hirth et al., Virus Research, 1991, Vol. 18, No. 2-3, pages 179-189, describes the determination and analysis of the nucleotide sequence of human papillomavirus type 41 (HPV-41).

Jacobs et al., Journal of Clinical Microbiology, 1997, Vol. 35, No. 3, pages 791-195, describes a PCR-EIA (enzyme immunoassay) procedure for the detection of fourteen high-risk HPV types and six low-risk HPV types.

Jacobs et al., Journal of Clinical Microbiology, 1995, Vol. 33, No. 4, pages 901-905, describes the use of cocktails of type-specific oligonucleotides for the hybridization of HPV general primer-mediated PCR (GP-PCR) products to distinguish between high-risk and low-risk HPV genotypes.

Schiffman et al., Journal of Clinical Microbiology, 1995, Vol. 33, No. 3, pages 545-550, describes an assessment of the reliability of a non-radioactive kit designed to detect 14 HPV types.

Seedorf et al., Virology, 1985, Vol. 145, pages 181-185, describes the complete sequence of HPV type 16 DNA cloned from an invasive cervical carcinoma.

Kennedy *et al.,* 1994, NCBI, Genebank database, Accession number K02718, describes the sense strand of the double-stranded circular genome of papilloma virus type 16 DNA.

In one aspect, the invention relates to a method for detecting HPV infection by high risk or low risk HPV types in individual cells by in situ hybridization.

In a related aspect, the invention relates to a reagent comprising a plurality of full length genomic probes to certain high risk HPV types or a second reagent comprising a plurality of full length genomic probes to certain low risk HPV types.

In a related aspect, the invention relates to exploiting the cross-reactivity of the probes according to the present invention to determine whether an HPV infected cell has any HPV type which is associated with malignancy not only those HPV types completely complementary to the probes.

In another aspect, the invention relates to methods correlating HPV type detection with the conventional cytology characterization as a second factor indicating whether the cells are normal or abnormal.

The present invention achieves these results by using a set of six essentially full length genomic probes in certain proportions which under low stringency cross react with all 13 currently known high risk HPV types and none of the low risk HPV types. The probes are labeled so that the labeling system may be visualized using conventional light microscopy which can also separately or simultaneously determine cell morphology as a measure of potential neoplasia. Alternatively, one may use a fluorescent labeling system and fluorescence microscopy to detect the results.

The protocol of the present invention was developed on Pap smears and ThinPrep samples. This system may also be applied for tissue sections. The samples may be previously stained or embedded in paraffin provided that they are destained or deparaffinized before use. Deparaffination may be performed by heat and detergent or solvent (e.g. xylene).

The probes being used are essentially full length genomic HPV probes specific for HPV types 16, 18, 31, 33, 35 and 51. The full length probes of the present invention have essentially the same sequence as given in GenBank Accession Numbers: K02718 - type 16; X05015 - type 18; J04353 - type 31; M62877 - type 51; M12732 and A12360 - type 33; M74117 - type 35. While some sequence variations and shortening of the probe length are permitted, these are still considered full length and are not similar to oligonucleotide probes as used in the prior art.

These probes range from roughly 6000 to 8000 base pairs each and are mixed together forming a reagent which is used with a hybridization cocktail using low stringency hybridization solution and conditions and lower stringency post wash solution and conditions. This permits one to detect the 13 known high risk types. The probe cocktail was tested on samples of known HPV types to be sure of detecting all high risk types with no cross hybridization to the low risk types. The results are provided in the attached manuscript and show a very good signal and low to no background.

The probes are labeled with digoxigenin or fluorescein by nick translation; however, a very wide variety of other labeling techniques may be used. Examples include, incorporation during PCR and random priming. The probes need not be labeled at all if one uses an anti-duplex or anti thymidine dimers antibody.

The in situ hybridization and detection according to the present invention has several advantages over other methods. First, since the present invention is an in situ hybridization, the cytology of the individual cell can be correlated with HPV positive or negative results. Second, since amplification of DNA by PCR, hybrid capture or other amplification techniques is not needed, the system is less subject to contamination or background yielding false positive or negative results. Third, the processed slides can be stored for years for later reference or confirmation. Fourth, since the reaction is performed on currently used patient sampling methods, no changes need to be instituted at the clinician level. A corollary to this advantage is that the reaction can be performed on destained Pap smears, so the patient does not need to return to the clinician's office for a second sampling in the event of an ambiguous Pap smear cytology. Fifth, the manipulation of the current probe set allows detection of currently classified and unclassified high risk HPV types. Alternatively, if a ThinPrep tube sampling was performed, the same cell suspension can be used to create a slide for the in-situ reaction. This allows the extra advantage that the system does not need reformulation to include newly identified HPV types. Sixth, one can differentiate between a few highly positive cells and many weakly positive ones to differentiate between a clinically important or subclinical infection, though ASCUS may be clinically important for reasons other than HPV.

The sample slide is processed through a series of steps to allow target DNA on the sample to hybridize to the probe without losing the morphology of the cell. See the method of PCT WO94/09022 and U.S. Patent Application 08/272,315, filed July 22, 1994. This includes a Proteinase K digestion and subsequent wash and dehydration steps. The digoxigenin labeled probe and target DNA are codenatured for 5 minutes at 95°C on a hot plate and then hybridization is allowed to proceed for three hours to overnight at 37°C. Following a post-hybridization wash, the slide is incubated with horseradish peroxidase (HRP) conjugated anti-digoxigenin antibody for 30 minutes at 37°C. The detection scheme utilizes the TrueBlue peroxidase substrate (KP Labs, Gaithersburg, MD). Alternatively, anti-digoxigenin antibody labeled with alkaline phosphatase with NBT/BCIP as chromogen was also used.

A thirty minute incubation of the TrueBlue substrate with the HRP detected probe produces a dark blue precipitate at the hybridization site. The cells were then counterstained with eosin, a pink cytoplasmic stain which contrasts well with the TrueBlue reaction product. The results are visualized using brightfield microscopy and can either be imaged or photographed. The slides can be stored indefinitely without losing signal. Elapsed time to set up the slide for hybridization is roughly 35 minutes and the post-hybridization elapsed time before the slide can be visualized is about 65 minutes.

Alternatively, one may use an automated protease digestion and subsequent wash steps. The probe and target DNA are codenatured for 5 minutes at 90°C on a hot plate and then hybridization is allowed to proceed for one hour to overnight at 37°C. Following a post-hybridization wash, the slide is incubated with alkaline phosphatase conjugated anti-digoxigenin antibody for 30 minutes at 37°C. The detection scheme utilizes an alkaline phosphatase substrate NBT/BCIP (Ventana Blue, Gaithersburg, MD). Alternatively, anti-fluorescein antibody labeled or subsequently conjugated with horseradish peroxidase with TMB as chromogen or alkaline phosphatase with NBT/BCIP as chromogen may also be used.

A thirty minute incubation of the Ventana Blue substrate with the alkaline phosphatase detected probe produces a blue/black precipitate at the hybridization site. The cells were then counterstained with eosin, a pink cytoplasmic stain which contrasts well with the NBT/BCIP reaction product. The results are visualized using brightfield microscopy and can either be imaged or photographed. The slides can be stored indefinitely without losing signal. Elapsed time to set up the slide for hybridization is roughly 25 minutes and the post-hybridization elapsed time before the slide can be visualized is about 90 minutes.

Brightfield detection is preferable to fluorescence because more laboratories have the necessary equipment, personnel are more familiar with the equipment and observation of cell morphology under brightfield detection, the analysis is easily automated and slides are readily preserved without signal loss. Other labeling systems, e.g. fluorescence, etc., is also an acceptable alternative.

The advantages of brightfield detection over fluorescent detection are important in this application. These slides could be preserved without signal loss due to fading, analysis could be more easily automated, and the morphology of the infected cells could be seen by the cytopathologists and cytotechnicians performing the test.

With a positive high risk HPV in situ result, the clinician may recommend another test every six months and may suggest a minor surgical treatment such as LEEP but is most likely to recommend colposcopy, probably with a biopsy.

The probes and reagents of the present invention are preferably packaged in kit form containing the probes mixed together as a single reagent in a container. Other reagents for sample treatment, hybridization and wash solutions, label detection systems and developing reagents for the label detection systems may also be incorporated in one or more other containers.

The following examples are included for purposes of illustrating certain aspects of the invention and should not be construed as limiting.

### EXAMPLE 1: PROBE SYNTHESIS AND ABILITIES

Six separate plasmids were prepared, one for each HPV type, with one plasmid containing the whole genome of a HPV type and the six types being types 16, 18, 31, 33, 35, and 51. The HPV was cloned into a plasmid by standard molecular biology techniques and are within the skill of the art. These plasmids were labeled by nick translation with digoxigenin dCTP. The labeled plasmids were then mixed together to form a single reagent. Incorporation of the digoxigenin nucleotide into the labeled DNA was verified by a dot-blot procedure.

The ability of each probe to cross-react with other high risk HPV types known to be associated with malignancy and not to cross react with low risk HPV types not associated with malignancy was tested. The data is given below in TABLE 1 where the probes used are listed as rows and the Pap smears ha e the HPV strain listed in the columns.

**TABLE 1**

| | 6 | 1 | 1 | 3 | 3 | 3 | 3 | 4 | 4 | 4 | 4 | 4 | 5 | 5 | 5 | 5 | 5 | 6 | 7 |
|---|---|---|---|---|---|---|---|---|---|---|---|---|---|---|---|---|---|---|---|
| | / | 6 | 8 | 1 | 3 | 5 | 9 | 1 | 2 | 3 | 4 | 5 | 1 | 2 | 6 | 8 | 9 | 8 | 0 |
| | 1 | | | | | | | | | | | | | | | | | | |
| | 1 | | | | | | | | | | | | | | | | | | |
| 1 | 2 | X | 0 | 1 | ½ | ½ | 0 | 0 | 1 | ½ | 1 | 0 | ½ | 0 | 0 | 1 | 0 | 0 | 0 |
| 6 | | | | | | | | | | | | | | | | | | | |
| 1 | 0 | 0 | X | 0 | 0 | 0 | 1 | ½ | 0 | 0 | 0 | 2 | 0 | 0 | ½ | 0 | 1 | 1 | 1 |
| 8 | | | | | | | | | | | | | | | | | | | |
| 3 | 1 | 1 | 0 | X | 1 | 2 | 0 | 0 | ½ | 0 | 0 | 0 | 1 | 0 | 1 | 1 | 0 | 0 | 0 |
| 1 | | | | | | | | | | | | | | | | | | | |
| 3 | 0 | ½ | 0 | 1 | X | ½ | 0 | 0 | 0 | 0 | 0 | ½ | 0 | 1 | 0 | 2 | 0 | 0 | 0 |
| 3 | | | | | | | | | | | | | | | | | | | |
| 3 | ½ | ½ | 0 | 1 | ½ | X | 0 | 0 | 0 | 0 | 0 | 0 | 0 | 2 | ½ | ½ | 0 | 0 | 0 |
| 5 | | | | | | | | | | | | | | | | | | | |
| 5 | 0 | ½ | 0 | 1 | 0 | 0 | 0 | 0 | 0 | 0 | 0 | 0 | X | 0 | 0 | 0 | 0 | 0 | 0 |
| 1 | | | | | | | | | | | | | | | | | | | |

| | | | | | | | | | | | | | | | | | | | |
|---|---|---|---|---|---|---|---|---|---|---|---|---|---|---|---|---|---|---|---|
| 0: No Detection ½: Barely Detected 1: Light Blue 2: Moderate Blue 3(X): Dark Blue | | | | | | | | | | | | | | | | | | | |

The probe to type 70 was not needed since all high risk HPV types are covered by other probes. Thus, it was not actually used in the reagent of the present invention.

Since some undesired cross reactivity was noted with probes to HPV type 16 and 31, the concentration of these two was lowered in the probe reagent to compensate. The percentages of each genomic probe in the DNA cocktail are given in TABLE 2.

**TABLE 2**

| HPV 16 | HPV 18 | HPV 31 | HPV 33 | HPV 35 | HPV 51 |
|---|---|---|---|---|---|
| 8.3% | 20.8% | 8.3% | 20.8% | 20.8% | 20.8% |

The resulting data was compared against 57 patient samples and resulted in the data of TABLE 3 where the cross reacting to the low risk types was essentially nullified. Comparison was made to another commercial HPV probe product.

**TABLE 3**

| HPV type | Digene Probe | Present Probe Cocktail |
|---|---|---|
| 2 | 3+ | 0 |
| 6/11 | 1+ | 0 |
| 6/11 | 1+ | 0 |
| 6/11 | 2+ | 0 |
| 6/11 | 3+ | 0 |
| 6/11 | 3+ | 1+ |
| 6/11 | 3+ | 0 |
| 6/11 | 3+ | 0 |
| 6/11 | 3+ | 0 |
| 6/11 | 3+ | 0 |
| 6/11 | 1+ | 0 |
| 6/11 | 1+ | 0 |
| 42 | 2+ | 0 |
| 43 | 2+ | 0 |
| 44 | 2+ | 0 |
| 16 | 1+ | 1+ |
| 16 | 1+ | 1+ |
| 16 | 2+ | 2+ |
| 16 | Weak | 1+ |
| 16 | 3+ | 3+ |
| 16 | 3+ | 3+ |
| 18 | 3+ | 3+ |
| 31 | 2+ | 2+ |
| 31 | 3+ | 3+ |
| 31 | 3+ | 3+ |
| 33 | 3+ | 3+ |
| 33 | 2+ | 2+ |
| 25 | 3+ | 3+ |
| 35 | 1+ | - |
| 51 | 3+ | 3+ |
| 30 | 3+ | 3+ |
| 30 | 3+ | 3+ |
| 30 | 3 | 3+ |
| 39 | 2 | 1+ |
| 39 | 2 | 2+ |
| 39 | 1 | Weak |
| 39 | 3 | 3+ |
| 39 | 3 | 3+ |
| 39 | 1 | 1+ |
| 45 | 3 | 3+ |
| 45 | 3 | 3+ |
| 52 | 3 | 3+ |
| 52 | 3 | 3+ |
| 52 | 2 | 2+ |
| 56 | 3 | 3+ |
| 56 | 1 | 1+ |
| 56 | 3 | 2+ |
| 56 | 3 | 1+ |
| 58 | 2 | 1+ |
| 59 | 2+ | 2+ |
| 68 | 1+ | 1+ |
| 70 | 3+ | 3+ |
| 70 | 2+ | 2+ |
| 70 | 1+ | 1+ |
| still novel | 2+ | 2+ |
| still novel | 1+ | 1+ |
| still novel | 1+ | 1+ |

As shown above, the present probe cocktail was shown to not give false positives with low risk HPV types unlike the probes of a competitor. Thus, the present invention will give a lower false positive reaction.

Furthermore, when both probe sets were applied to normal Pap smears containing HPV, the Digene probes indicated a positive reaction whereas the coctail of the present invention did not yield a strong positive reaction. This is an important check as occasionally, Pap smears are misread. It is generally thought that ten percent or more of the population is infected with HPV but this only poses a risk in the presence of displasia or other morphological abnormality. Again, the present invention yields fewer false positives.

### EXAMPLE 2: SAMPLE PREPARATION AND PRETREATMENT

Uterine cervix cells were sampled and smeared to form a conventional Pap smear or suspended in PreservCyt (Cytyc Corporation), a buffered fixative and preservative solution. The ThinPrep 2000 (Cytyc Corporation) was used in make two ThinPrep slides for each patient. One slide was stained for conventional cytology similar to that of conventional PAP smears and the other slide was prepared as below.

A variety of protease treatments were attempted. The effect of varying the concentrations of proteinase K and pepsin on the intensity of the in situ hybridization signal with an alu probe on archival, destained Pap smears results are given in TABLE 4. Archived Pap smears were deparaffinized by submersion in xylene and washed in ethanol. Destaining was accomplished by washing the slides for 20 minutes at room temperature in 70% ethanol and 0.1 N HCl. The slides were then washed in tap water for 10 minutes, rinsed in 100% ethanol for 5 minutes, and air dried. All samples were digested for 20 minutes at 37°C. The post hybridization wash was done at high stringency (60°C, 0.2X SSC and 2% BSA. The following procedure was used on patient samples.

**TABLE 4**

| Protease | 0 | 1 | 10 | 25 | 50 | 100 | 200 | 2,000 |
|---|---|---|---|---|---|---|---|---|
| Concentration | (µg/ml) | | | | | | | |
| Proteinase K | | | | | | | | |
| in water | +/- | 1+ | 3+ | 3+ | 3+ | 2+ | 1+ | OD |
| in 1XSSC | +/- | 3+ | 1+ | OD | OD | OD | OD | OD |

| Pepsin | | | | | | | | |
|---|---|---|---|---|---|---|---|---|
| in water | +/- | 1+ | 2+ | 2+ | 3+ | 3+ | 2+ | OD |
| in 0.1 N HCl | +/- | 0 | 1+ | 1+ | 2+ | 2+ | 3+ | OD |

| | | | | | | | | |
|---|---|---|---|---|---|---|---|---|
| signal intensity was scored by the percentage of positive cells (,25%=1+, 25-50%=2+, >50%=3+). OD means overdigested, in which cell morphology is poor and no signal is evident. | | | | | | | | |

The Pap smear and the ThinPrep slide were incubated for 20 minutes at 37°C in a solution of 10 micrograms per milliliter of Proteinase K in 2X SSC. Following the incubation, the slide was washed for 2 minutes at room temperature in 2X SSC, dehydrated in a series of 70%, 80%, and 95% room temperature ethanol solutions for 1 minute each and air dried.

### EXAMPLE 3: PROBE AND TARGET HYBRIDIZATION

A probe solution was made consisting of 0.5 nanograms per milliliter of HPV types 18, 33, 35, and 51 and 0.2 nanograms per milliliter of HPV types 16 and 31 in Hybrisol IX (Ventana) using the probes prepared above. Ten microliters of this probe solution was pipetted onto the sample slide and the specimen was covered with a 22 mm round coverslip and optionally sealed with rubber cement. The slide was placed on a prewarmed 95°C hot plate for 5 minutes to denature the probe and target DNA and then transferred to a humidified chamber and placed in a 37°C incubator. The slide was incubated at 37°C in the humidified chamber for 2 to 16 hours to hybridize. After the 37°C incubation for hybridization, the rubber cement and coverslip are removed. The slide is washed and the signal detected.

Both high stringency post hybridization wash conditions (0.2X SSC, 2% BSA, 60°C, 10 minutes) and low stringency post hybridization wash conditions (2X SSC, 2% BSA, 45°C, 10 minutes) were used on a number of patient samples for whom the HPV type was determined. The data is given in TABLE 5 where detection of different HPV types in cervical biopsies by the high risk HPV consensus probe as a function of stringency of the post hybridization wash.

**TABLE 5**

| HPV DNA POSITIVE | | |
|---|---|---|
| HPV type | Low Stringency | High Stringency |
| Common HPV types | | |
| 6/11 | 0/11 | 0/11 |
| 16/18 | 11/11 | 11/11 |
| 31/33/35 | 7/7 | 7/7 |
| 51 | 4/4 | 4/4 |

| Rare HPV types | | |
|---|---|---|
| 42/43/44 | 0/3 | 0/3 |
| 30 | 3/3 | 1/3 |
| 39 | 7/7 | 2/7 |
| 45 | 3/3 | 3/3 |
| 52 | 3/3 | 1/3 |
| 56 | 4/4 | 1/4 |
| 58/59/68 | 3/3 | 1/3 |
| 70 | 3/3 | 3/3 |

| | | |
|---|---|---|
| Low stringency is a post hybridization wash of 10 minutes in 2X SSC with 2% BSA at 45°C. High stringency is a post hybridization wash of 10 minutes in 0.2X SSC with 2% BSA at 60°C. | | |

These common HPV types listed comprise about 75% of those types found in lower grade cervical SILs (4-6). Each of the rare HPV types is found in from 1-3% of low grade cervical SILs (4-6). Note that all of the patient samples with high risk HPV types associated with malignancy were detected under low stringency and none of the low risk HPV types not associated with malignancy were detected under low stringency.

### EXAMPLE 4: IMMUNOCHEMICAL DETECTION

The sample on the slide was then incubated with horseradish peroxidase (HRP) labeled anti-digoxigenin antibody (Boehrigher Mannheim GMBH). The slide was washed three times for two minutes each in 1X PBD to remove any unbound or loosely bound antibody. The slide was removed from 1X PBD and allowed to drain briefly. Two hundred microliters of TrueBlue Peroxidase Substrate^{™} (KPL) was added to the slide and the reaction proceeds at room temperature for three minutes. The slide is rinsed in distilled water and allowed to air dry.

The slide was dipped in a 1/4X solution of Eosin in ethanol to counterstain. The slide was rinsed three times in distilled water and allowed to air dry. To mount, the slide was dipped in xylenes and a drop of Permount (Fisher) is added. The slide is then covered with a 22 mm round glass coverslip.

Cells with high risk HPV integrated in the cell demonstrate a blue precipitate in the nuclei with minimal slide background. The cytoplasm is counterstained pink for contrast. Cellular morphology confirms that high risk HPV types were was present in a cell which would be classified as abnormal and normal cells lack any positive signal.

Alternatively, alkaline phosphatase labeled anti-digoxigenin antibody may be used along with NBT/BCIP detection reagents as is used in the attached manuscript.

### EXAMPLE 5: COMPARISON OF PAP SMEAR RESULTS TO HPV TYPE TESTING

A sizable number of normal, ASCUS and SIL PAP smears were tested for HPV type status. Those detected by the probe reagent of the present invention are indicated as positive cases. The detection of HPV DNA using the high risk consensus probe at low stringency conditions as a function of the cytologic diagnosis and, for cases of ASCUS, clinical follow up data is given in TABLE 6.

**TABLE 6**

| Pap Smear Result | HPV Positive | |
|---|---|---|
| Cases | | |
| Normal | 1/19 | (5%) |
| ASCUS (total) | 16/40 | (40%) |
| SIL | 18/23 | (78%) |
| | | |
| ASCUS (biopsy of SIL within 6 months) | 14/21 | (67%) |
| ASCUS (biopsy negative for SIL within 6 months) | 2/19 | (10%) |

The one normal positive was rescreened by two cytotechnologists who did not know the HPV result and was classified by each as ASCUS. The ASCUS subgrouping was statistically significant difference at p=0.05 using the nonparametric two-tailed Mann-Whitney test as per InStat, Version 2.0 (GraphPad Software, San Diego, CA).

### EXAMPLE 6: TESTING CELL LINES

The probes above were tested against three known cell lines to confirm their ability to detect HPV with respect to the copy number of viruses in each cell line obtained from ATCC. The data is given in TABLE 7.

**TABLE 7**

| Cell Line | HPV type | Copy number | Detected |
|---|---|---|---|
| SiHa | 16 | 1 | ✔ |
| HeLa | 18 | 20 | ✔✔ |
| CaSki | 16 | 600 | ✔✔ |

It will be understood that various modifications may be made to the embodiments disclosed herein. Therefore, the above description should not be construed as limiting, but merely as exemplifications of preferred embodiments. Those skilled in the art will envision other modifications within the scope and spirit of the claims appended hereto.

## Claims

1. A reagent for detecting human papilloma virus (HPV) DNA in a cell sample, which indicates the patient providing the cell sample is at risk for cancer, comprising a plurality of genomic HPV DNA probes; wherein:
(a) a first genomic HPV DNA probe comprises a plurality of nucleic acid fragments that detectably hybridize to essentially the full-length genomic sequence of HPV type 16,
(b) a second genomic HPV DNA probe comprises a plurality of nucleic acid fragments that detectably hybridize to essentially the full-length genomic sequence of HPV type 18,
(c) a third genomic HPV DNA probe comprises a plurality of nucleic acid fragments that detectably hybridize to essentially the full-length genomic sequence of HPV type 31,
(d) a fourth genomic HPV DNA probe comprises a plurality of nucleic acid fragments that detectably hybridize to essentially the full-length genomic sequence of HPV type 33,
(e) a fifth genomic HPV DNA probe comprises a plurality of nucleic acid fragments that detectably hybridize to essentially the full-length genomic sequence of HPV type 35, and
(f) a sixth genomic HPV DNA probe comprises a plurality of nucleic acid fragments that detectably hybridize to essentially the full-length genomic sequence of HPV type 51;
wherein the proportion of total HPV DNA in the reagent that comprises nucleic acid fragments of the first genomic HPV DNA probe and the proportion of total HPV DNA in the reagent that comprises nucleic acid fragments of the third genomic HPV DNA probe are decreased relative to the proportions of the total HPV DNA in the reagent that comprises nucleic acid fragments of the other HPV DNA probes;
and wherein the nucleic acid fragments of the genomic HPV DNA probes do not detectably hybridize to the genomic sequence of a low-risk HPV type.

2. The reagent of claim 1, wherein the nucleic acid fragments of the genomic HPV DNA probe sets also hybridize to the genomic sequence of HPV types 39, 45, 52, 56, 58, 59, 68 and 70.

3. The reagent of either claim 1 or 2, wherein the cell sample is cervical cells taken from a patient.

4. The reagent of claim 1, wherein:
(a) the plurality of nucleic acid fragments of the first genomic HPV DNA probe constitute about 8.3% of the total HPV DNA in the reagent,
(b) the plurality of nucleic acid fragments of the second genomic HPV DNA probe constitute about 20.8% of the total HPV DNA in the reagent,
(c) the plurality of nucleic acid fragments of the third genomic HPV DNA probe constitute about 8.3% of the total HPV DNA in the reagent,
(d) the plurality of nucleic acid fragments of the fourth genomic HPV DNA probe constitute about 20.8% of the total HPV DNA in the reagent,
(e) the plurality of nucleic acid fragments of the fifth genomic HPV DNA probe constitute about 20.8% of the total HPV DNA in the reagent, and
(f) the plurality of nucleic acid fragments of the sixth genomic HPV DNA probe constitute about 20.8% of the total HPV DNA in the reagent.

5. A method for detecting human papilloma virus (HPV) DNA in a cell sample which indicates the patient providing the cell sample is at risk for cancer comprising.
(a) adding the reagent of any one of claims 1 to 4 under hybridization conditions, and
(b) detecting the presence or absence of hybridization inside cells in the cell sample.

6. The method of claim 5 further comprising pretreating the cell sample with a protease.

7. The method of any one claims 5 or 6 further comprising destaining and/or deparaffining the cell sample.

8. The method of any one of claims 5 to 7 wherein the cell sample contains abnormal cervical cells.

9. A kit for detecting human papilloma virus DNA in a sample comprising a container containing the reagent of any one of claims 1 to 4.

## Patentansprüche

1. Reagenz zum Nachweisen von humaner Papilloma-Virus (HPV)-DNA in einer Zell-Probe, welches anzeigt, ob der die Zell-Probe bereitstellende Patient einem Krebsrisiko unterliegt, umfassend eine Vielzahl genomischer HPV DNA-Sonden, wobei:
(a) eine erste genomische HPV DNA-Sonde eine Vielzahl von Nukleinsäurefragmenten umfasst, welche nachweisbar mit der im Wesentlichen vollen Länge der genomischen Sequenz von HPV Typ 16 hybridisieren,
(b) eine zweite genomische HPV DNA-Sonde eine Vielzahl von Nukleinsäurefragmenten umfasst, welche nachweisbar mit der im Wesentlichen vollen Länge der genomischen Sequenz von HPV Typ 18 hybridisieren,
(c) eine dritte genomische HPV DNA-Sonde eine Vielzahl von Nukleinsäurefragmenten umfasst, welche nachweisbar mit der im Wesentlichen vollen Länge der genomischen Sequenz von HPV Typ 31 hybridisieren,
(d) eine vierte genomische HPV DNA-Sonde eine Vielzahl von Nukleinsäurefragmenten umfasst, welche nachweisbar mit der im Wesentlichen vollen Länge der genomischen Sequenz von HPV Typ 33 hybridisieren,
(e) eine fünfte genomische HPV DNA-Sonde eine Vielzahl von Nukleinsäurefragmenten umfasst, welche nachweisbar mit der im Wesentlichen vollen Länge der genomischen Sequenz von HPV Typ 35 hybridisieren, und
(f) eine sechste genomische HPV DNA-Sonde eine Vielzahl von Nukleinsäurefragmenten umfasst, welche nachweisbar mit der im Wesentlichen vollen Länge der genomischen Sequenz von HPV Typ 51 hybridisieren,
wobei das Verhältnis der gesamten HPV DNA im Reagenz, welche Nukleinsäurefragmente der ersten genomischen HPV DNA-Sonde umfasst, und das Verhältnis der gesamten HPV DNA im Reagenz, welche Nukleinsäurefragmente der dritten genomischen HPV DNA-Sonde umfasst, relativ zu den Verhältnissen der gesamten HPV DNA im Reagenz, welche Nukleinsäurefragmente der anderen HPV DNA-Sonden umfasst, verringert ist,
und wobei die Nukleinsäurefragmente der genomischen HPV DNA-Sonden nicht nachweisbar mit der genomischen Sequenz eines HPV-Typs niedrigen Risikos hybridisieren.

2. Reagenz nach Anspruch 1, wobei die Nukleinsäurefragmente der genomischen HPV DNA-Sondensätze auch mit der genomischen Sequenz der HPV-Typen 39, 45, 52, 56, 58, 59, 68 und 70 hybridisieren.

3. Reagenz nach entweder Anspruch 1 oder 2, wobei die Zell-Probe Gebärmutterhalszellen ist, welche von einem Patienten entnommen sind.

4. Reagenz nach Anspruch 1, wobei
(a) die Vielzahl von Nukleinsäurefragmenten der ersten genomischen HPV DNA-Sonde etwa 8,3% der gesamten HPV DNA im Reagenz ausmacht,
(b) die Vielzahl von Nukleinsäurefragmenten der zweiten genomischen HPV DNA-Sonde etwa 20,8% der gesamten HPV DNA im Reagenz ausmacht,
(c) die Vielzahl von Nukleinsäurefragmenten der dritten genomischen HPV DNA-Sonde etwa 8,3% der gesamten HPV DNA im Reagenz ausmacht,
(d) die Vielzahl von Nukleinsäurefragmenten der vierten genomischen HPV DNA-Sonde etwa 20,8% der gesamten HPV DNA im Reagenz ausmacht,
(e) die Vielzahl von Nukleinsäurefragmenten der fünften genomischen HPV DNA-Sonde etwa 20,8% der gesamten HPV DNA im Reagenz ausmacht, und
(f) die Vielzahl von Nukleinsäurefragmenten der sechsten genomischen HPV DNA-Sonde etwa 20,8% der gesamten HPV DNA im Reagenz ausmacht.

5. Verfahren zum Nachweisen von humaner Papilloma-Virus (HPV)-DNA in einer Zellprobe, welches anzeigt, ob der die Zellprobe bereitstellende Patient einem Krebsrisiko unterliegt, umfassend:
(a) das Hinzugeben des Reagenzes nach einem der Ansprüche 1 bis 4 unter Hybridisierungsbedingungen, und
(b) das Nachweisen der Anwesenheit oder Abwesenheit von Hybridisierung in Zellen in der Zellprobe.

6. Verfahren nach Anspruch 5, weiter umfassend das Vorbehandeln der Zellprobe mit einer Protease.

7. Verfahren nach einem der Ansprüche 5 oder 6, weiter umfassend das Entfärben und/oder Entparaffinieren der Zellprobe.

8. Verfahren nach einem der Ansprüche 5 bis 7, wobei die Zellprobe abnormale Gebärmutterhalszellen umfasst.

9. Kit zum Nachweisen von humaner Papilloma-Virus-DNA in einer Probe, umfassend einen Behälter, welcher das Reagenz nach einem der Ansprüche 1 bis 4 enthält.

## Revendications

1. Réactif destiné à détecter l'ADN du virus du papillome humain (VPH) dans un échantillon cellulaire, lequel indique que le patient fournissant l'échantillon cellulaire risque d'avoir le cancer, qui comprend une pluralité de sondes à ADN génomique du VPH ; dans lequel :
(a) une première sonde à ADN génomique du VPH comprend une pluralité de fragments d'acides nucléiques qui s'hybrident de manière détectable avec essentiellement la longueur complète de la séquence génomique du VPH de type 16,
(b) une deuxième sonde à ADN génomique du VPH comprend une pluralité de fragments d'acides nucléiques qui s'hybrident de manière détectable avec essentiellement la longueur complète de la séquence génomique du VPH de type 18,
(c) une troisième sonde à ADN génomique du VPH comprend une pluralité de fragments d'acides nucléiques qui s'hybrident de manière détectable avec essentiellement la longueur complète de la séquence génomique du VPH de type 31,
(d) une quatrième sonde à ADN génomique du VPH comprend une pluralité de fragments d'acides nucléiques qui s'hybrident de manière détectable avec essentiellement la longueur complète de la séquence génomique du VPH de type 33,
(e) une cinquième sonde à ADN génomique du VPH comprend une pluralité de fragments d'acides nucléiques qui s'hybrident de manière détectable avec essentiellement la longueur complète de la séquence génomique du VPH de type 35, et
(f) une sixième sonde à ADN génomique du VPH comprend une pluralité de fragments d'acides nucléiques qui s'hybrident de manière détectable avec essentiellement la longueur complète de la séquence génomique du VPH de type 51 ;
dans lequel la proportion de l'ADN total du VPH dans le réactif qui comprend les fragments d'acides nucléiques de la première sonde à ADN génomique du VPH et la proportion de l'ADN total du VPH dans le réactif qui comprend les fragments d'acides nucléiques de la troisième sonde à ADN génomique du VPH sont diminuées relativement aux proportions de l'ADN total du VPH dans le réactif qui comprend les fragments d'acides nucléiques des autres sondes à ADN du VPH ;
et dans lequel les fragments d'acides nucléiques des sondes à ADN génomique du VPH ne s'hybrident pas de manière détectable avec la séquence génomique d'un type de VPH à faible risque.

2. Réactif selon la revendication 1, dans lequel les fragments d'acides nucléiques des sets de sondes à ADN génomique du VPH s'hybrident également à la séquence génomique des VPH de type 39, 45, 52, 56, 58, 59, 68 et 70.

3. Réactif selon l'une quelconque des revendications 1 ou 2, dans lequel l'échantillon cellulaire est des cellules cervicales prélevées chez un patient.

4. Réactif selon la revendication 1, dans lequel :
(a) la pluralité de fragments d'acides nucléiques de la première sonde à ADN génomique du VPH constitue environ 8,3 % de l'ADN total du VPH dans le réactif,
(b) la pluralité de fragments d'acides nucléiques de la deuxième sonde à ADN génomique du VPH constitue environ 20,8 % de l'ADN total du VPH dans le réactif,
(c) la pluralité de fragments d'acides nucléiques de la troisième sonde à ADN génomique du VPH constitue environ 8,3 % de l'ADN total du VPH dans le réactif,
(d) la pluralité de fragments d'acides nucléiques de la quatrième sonde à ADN génomique du VPH constitue environ 20,8 % de l'ADN total du VPH dans le réactif,
(e) la pluralité de fragments d'acides nucléiques de la cinquième sonde à ADN génomique du VPH constitue environ 20,8 % de l'ADN total du VPH dans le réactif, et
(f) la pluralité de fragments d'acides nucléiques de la sixième sonde à ADN génomique du VPH constitue environ 20,8 % de l'ADN total du VPH dans le réactif.

5. Procédé destiné à détecter l'ADN du virus du papillome humain (VPH) dans un échantillon cellulaire, lequel indique que le patient fournissant l'échantillon cellulaire risque d'avoir le cancer qui comprend :
(a) l'ajout du réactif selon l'une quelconque des revendications 1 à 4 dans des conditions d'hybridation, et
(b) la détection de la présence ou de l'absence d'hybridation à l'intérieur des cellules dans l'échantillon cellulaire.

6. Procédé selon la revendication 5 qui comprend en outre le prétraitement de l'échantillon cellulaire avec une protéase.

7. Procédé selon l'une quelconque des revendications 5 ou 6 qui comprend en outre la décoloration et / ou le déparaffinage de l'échantillon cellulaire.

8. Procédé selon l'une quelconque des revendications 5 à 7 dans lequel l'échantillon cellulaire contient des cellules cervicales anormales.

9. Kit destiné à détecter l'ADN du virus du papillome humain dans un échantillon qui comprend un conteneur contenant le réactif selon l'une quelconque des revendications 1 à 4.
